# EUROPEAN PATENT APPLICATION

(11) **EP 0 716 848 A2**
(43) Date of publication of application: **19.06.1996**
(21) Application number: 95307685.8
(22) Date of filing: 27.10.1995
(51) Int. Cl.: A61K 7/48

(54) **Cosmetic emulsions with a deposition triggering lipid system**

(30) Priority: 02.12.1994 US 348382
(71) Applicant: UNILEVER PLC, London EC4P 4BQ (GB); UNILEVER N.V., NL-3013 AL Rotterdam (NL)
(72) Inventor: Znaiden, Alexander, Trumbull, Connecticut 06611 (US); Cheney, Michael Charles, Fairfield, Connecticut 06430 (US); Ziegler, Philip Dale, Oxford, Connecticut 06478 (US)
(74) Representative: Evans, Jacqueline Gail Victoria

(57) **Abstract**

An oil and water cosmetic emulsion is provided including isoparaffin, a sterol and a phosphatide. The combination of sterol and phosphatide effects the deposition of isoparaffin and other components resulting in a quick absorbing, less greasy and long lasting conditioned feel onto the skin.

## Description

### BACKGROUND OF THE INVENTION

### Field of the Invention

The invention concerns oil and water cosmetic emulsions formulated with a lipid system that triggers deposition of emollients and oils onto the skin.

### The Related Art

Petroleum jelly, commercially available under the Vaseline® brand, has long been recognized as therapeutically effective against dry skin. A major negative limiting use of petroleum jelly is the greasiness of this material. Petroleum jelly operates as a barrier but does not penetrate into the skin. Transfer of this material onto clothing, therefore, readily occurs. By contrast, water-based formulations, although often less effective, do not transfer to clothing and exhibit better feel properties. Not surprisingly, water-based cosmetic compositions such as aqueous lotions and creams have garnered the main share of the market.

An approach to providing the benefits of petroleum jelly while neutralizing its greasy feel has been the preparation of aqueous petroleum jelly emulsions. Emulsifiers have been utilized to provide compatibility between aqueous and oil phases. Attempts at emulsification have not always been successful. Even when successful, the resultant product often fails to exhibit the skin protective properties of petroleum jelly. New and improved emulsifying systems would be highly desirable.

Illustrative of the cosmetic emulsion art is U.S. Patent 4,760,096 (Sakai et al.) which discloses a skin moisturizing preparation that includes a phosphatide, at least one C₁₀-C₃₀ carboxylic acid sterol ester and at least one C₆-C₁₂ alkanoic triglyceride in a dermatologically acceptable carrier. WO 90/01323 (Bernstein) describes a composition for preventing dry skin based on a lipid concentrate combining three naturally-occurring lipid groups found in the Stratum corneum. These groups include fatty acids, sterols (e.g. cholesterol) and sterol esters, and phospholipids and glycolipids (e.g. lecithin and ceramides). U.S. Patent 4,855,090 (Wallach) approaches the problem through the use of liposome technology. A nonaqueous lipophilic phase is combined with an aqueous phase under high shear mixing conditions to form the liposomes. Among the components included in the lipophilic phase are cholesterol and polyoxyethylene fatty ether surfactant while the aqueous phase contains phosphatides such as lecithin.

Dermasil®, sold by the Chesebrough-Pond's Company, containing relatively high levels of petroleum jelly in an aqueous emulsion represents another advance in this area. Stabilization is achieved by a combination of sterol, lecithin and sunflower seed oil. A description of this formulation is found in U.S. Patent 5,310,556 (Ziegler).

Even when properly emulsified, petroleum jelly and also mineral oil impart a perceived heaviness to a cosmetic product. Isoparaffinic hydrocarbons have been identified in the literature as useful substitutes for petroleum jelly and mineral oil. Consumers appreciate the less heavy feel of the isoparaffinic hydrocarbons yet are provided with the same lubricity characteristics. In the marketplace, Vaseline® Intensive Care® Lotion has utilized isoparaffin technology delivered through an oil-in-water system. A description of this formulation is found in U.S. Patent 4,939,179 (Cheney et al.).

Research has continued to seek products combining the benefits of an occlusives depositing product such as Dermasil® with that of an aesthetically preferred lighter emulsion such as found in Vaseline® Intensive Care® lotion.

Accordingly, it is an object of the present invention to provide a cosmetic composition for skin which avoids any perceived feeling of heaviness while imparting no less protection against dry skin condition.

Another object of the present invention is to provide a cosmetic composition for skin which is non-greasy.

Still another object of the present invention is to provide a cosmetic composition for skin in emulsion form having adequate storage stability.

Still a further object of the present invention is to provide a cosmetic composition for skin which promotes deposition of emollients and oils from emulsion that can counteract at least the appearance of dry skin.

Yet a further object of the present invention is to provide a cosmetic emulsion that is quick absorbing, less greasy and imparts a long lasting conditioned feel to the skin.

These and other objects of the present invention will more fully be appreciated through the detailed description that follows.

### SUMMARY OF THE INVENTION

An oil and water cosmetic emulsion is provided that includes the following components:
(i) from about 0.1 to 20% of isoparaffin;
(ii) from about 0.001 to 0.8% of sterol; and
(iii) from about 0.001 to 0.8% of phosphatide.

### DETAILED DESCRIPTION OF THE INVENTION

Now it has been found that oil and water emulsions incorporating isoparaffin as the essential hydrocarbon occlusive agent, when formulated with a relatively small amount of lipid system triggers deposition of lubricious additives onto skin upon rub-in.
More specifically, it has been discovered that a lipid system formed of sterol and phosphatide at combined levels of less than 0.5% trigger deposition. Combined levels of 1% or higher were found counter-effective in achieving deposition.

Accordingly, an essential element of the present invention is an isoparaffin. Isoparaffins are largely saturated aliphatic hydrocarbons with highly branched structures having from 11 to 13 carbon atoms per molecule. Preferably the isoparaffins are selected from one or a mixture of isomeric species including:

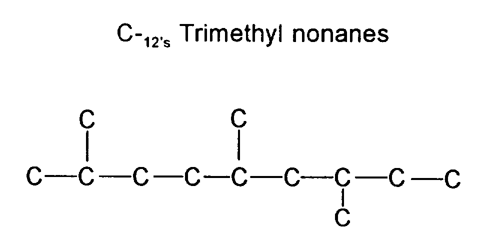

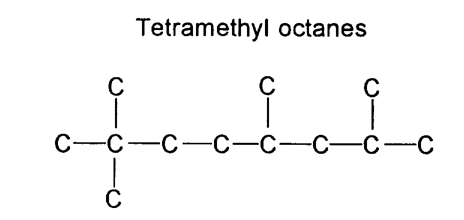

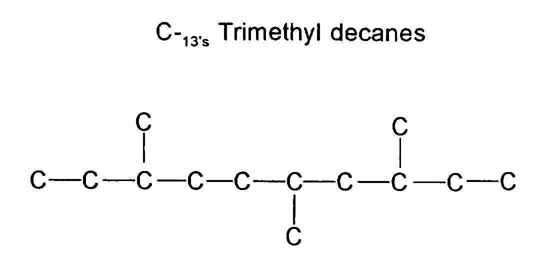

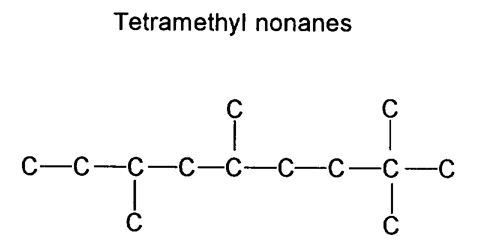

A particularly preferred type of isoparaffin is a material known as Isopar L, sold by the Exxon Company, Houston, Texas. The material is a clear, odorless and colorless thin liquid with a flash point of 142°F. Isopar L has an average molecular weight of 171 and a viscosity of 2.6 centistokes at 15.5°C.

Isoparaffin normally will be incorporated into the compositions of this invention in an amount from about 0.1 to about 20% by weight of the total. Preferably this material will be present in an amount from about 0.5% to about 10%, optimally from about 1 to about 3% by weight of the total composition.

A second essential element of the present invention is a sterol. Preferably the sterol is a β-sterol having a tail on the 17 position and having no polar groups. Illustrative of this category is cholesterol, sitosterol, stigmasterol and ergosterol. Cholesterol and soy sterol are preferred. A commercial source of soy sterol is a product known as Generol 122®, available from the Henkel Corporation, Ambler, Pa. Generol 122® is a mixture of stigmasterol, sitosterol and ergosterol. Cosmetic compositions according to the present invention will include the sterol in an amount from about 0.001 to 0.8%, preferably from about 0.05 to about 0.5%, optimally from about 0.10 to about 0.3% by weight.

A further essential component according to the present invention is a phosphatide. Examples of suitable phosphatides are lecithin, phoshatidyl choline, phosphatidyl ethanolamine, phosphatidyl serine, phosphatidyl inositol, diphosphatidyl glycerol and mixtures thereof. Lysophosphoglycerides may also serve as the phosphatide. Preferred from among the foregoing list is lecithin. Amounts of the phosphatide will range from about 0.001 to 0.8%, preferably from about 0.01 to about 0.5%, optimally from about 0.10 to about 0.3% by weight.

Relative ratios of sterol and phosphatide may range from about 20:1 to about 1:20, preferably from about 5:1 to about 1:1, optimally about 2:1. Total level of sterol and phosphatide should range from about 0.01 to 0.9%, preferably from about 0.05 to about 0.8%, more preferably from about 0.1 to about 0.6%, optimally from about 0.2 to about 0.5% by weight of the cosmetic compositions.

Advantageously, compositions of the present invention will also contain an alkyl phosphate salt, more particularly a C₈-C₂₂ alkyl phosphate salt. Illustrative phosphate salts include salts of cetyl phosphate, myristyl phosphate, lauryl phosphate and stearyl phosphate. Salt counterions may be selected from alkalimetal, ammonium, trialkylammonium and di- or tri- alkanolammonium cations. Particularly preferred are triethanolammonium (TEA) and diethanolammonium (DEA) cetyl phosphates, commercially available under the trademark Amphisol from the Givaudan Corporation. The amount of alkyl phosphate salt will range from about 0.01% to about 10% by weight of the total compositon. Particularly preferred is a concentration from about 0.25% to about 1%, optimally between about 0.25% and 0.5% by weight of the total composition.

A variety of emollient components may desirably be formulated within the oily phase of the composition. These substances include triglycerides, fatty acids and alcohols and certain types of esters. Ordinarily petroleum jelly will be present at levels no higher than about 0.5%, and usually even absent from the composition.

Among the triglycerides, most preferred for the present invention are the C₁₆-C₂₂ alkanoic triglycerides. Preferably the triglyceride will be based on a material whose major component is linoleic acid residues. Sunflower seed oil is the preferred embodiment. Amounts of the triglyceride will range from about 0.1 to about 20%, preferably from about 0.5 to 5%, optimally from about 1 to about 3% by weight of the total composition.

Fatty acids having from 10 to 22 carbon atoms can usefully be employed as emollients for the present invention. Illustrative are stearic acid, palmitic acid, myristic acid, lauric acid and oleic acid. Levels of these emollients may range from about 0.5 to about 10%, preferably from about 1 to about 5%, optimally from about 2 to about 3% by weight of the total composition.

Fatty alcohols having from 10 to 22 carbon atoms may also be used as emollients. Illustrative are cetyl alcohol, myristyl alcohol, stearyl alcohol and oleoyl alcohol. Most preferred is cetyl alcohol. Levels of these emollients may range from about 0.01 to about 10%, preferably from about 0.1 to about 5%, optimally from about 0.3 to about 1% by weight of the total composition.

Certain types of esters may be incorporated both for emollient and emulsification purposes. Particularly preferred are C₁₀-C₂₂ fatty acid monoglycerides such as glyceryl monostearate. Amounts of this material may range from about 0.05 to about 10%, preferably from about 0.1 to about 2%, optimally from about 0.5 to about 1% by weight of the total composition.

In addition to the aforementioned components, the composition may also include from about 0.1 to about 20% each of the following: a wax (e.g. carnauba or microcrystalline polyethylene wax), a lanolin derived material (e.g. Acetulan which is a 9:1 mixture of cetyl acetate and acetyl lanolin alcohol available from Amerchol), glycol stearate, stearamide AMP and mixtures of these ingredients.

There may also be present from about 0.01 to 2% of adjunct water-phase thickening agents such as Veegum®, a magnesium aluminum silicate sold by the Vanderbilt Chemical Co., and Carbopol® 934, a cross-linked polyacrylate polymer available from the B.F. Goodrich Co. Proteins may be included to improve skin feel. Other minor but important functional ingredients such as colorant, fragrance and preservative are normally also included, each present in an effective amount to accomplish its function. Suitable preservatives include the methyl and propyl parabens and sodium ethylenediaminetetraacetates.

Of course, water is an essential component of emulsions according to the present invention. The amount of water will range from about 50 to about 95%, preferably from about 75% to about 90% by weight of the total composition.

Humectants of the polyhydric alcohol-type may also be included in the compositions of this invention. The humectant aids in increasing the effectiveness of the emollient, reduces scaling, stimulates removal of built-up scale and improves skin feel. Typical polyhydric alcohols include polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, 1,2,6-hexanetriol, ethoxylated glycerin, propoxylated glycerin and mixtures thereof. For best results the humectant is preferably glycerin. The amount of humectant may range anywhere from 0.5 to 20%, preferably between 1 and 15% by weight of the total composition.

For improved lubricity, there may also be included one or more silicone oils or fluids which may be selected from a dimethyl polysiloxane, a methylphenyl polysiloxane and an alcohol-soluble silicone glycol copolymer. Preferred siloxanes include dimethyl polysiloxane (CTFA name: dimethicone), a polysiloxane endblocked with trimethyl units and polydimethylcyclosiloxane, (CTFA name: cyclomethicone). The preferred siloxanes exhibit a viscosity from about 2 to 50 centistokes at 25°C. Amounts of the silicones can range from about 0.01 to about 20%, preferably from about 0.1 to about 1%, optimally from about 0.2 to about 0.5% by weight of the total composition.

The following examples will more fully illustrate the embodiments of this invention. All parts, percentages and proportions referred to herein and in the appended claims are by weight unless otherwise indicated.

### EXAMPLES 1-10

The following formulations are representative of the present invention.

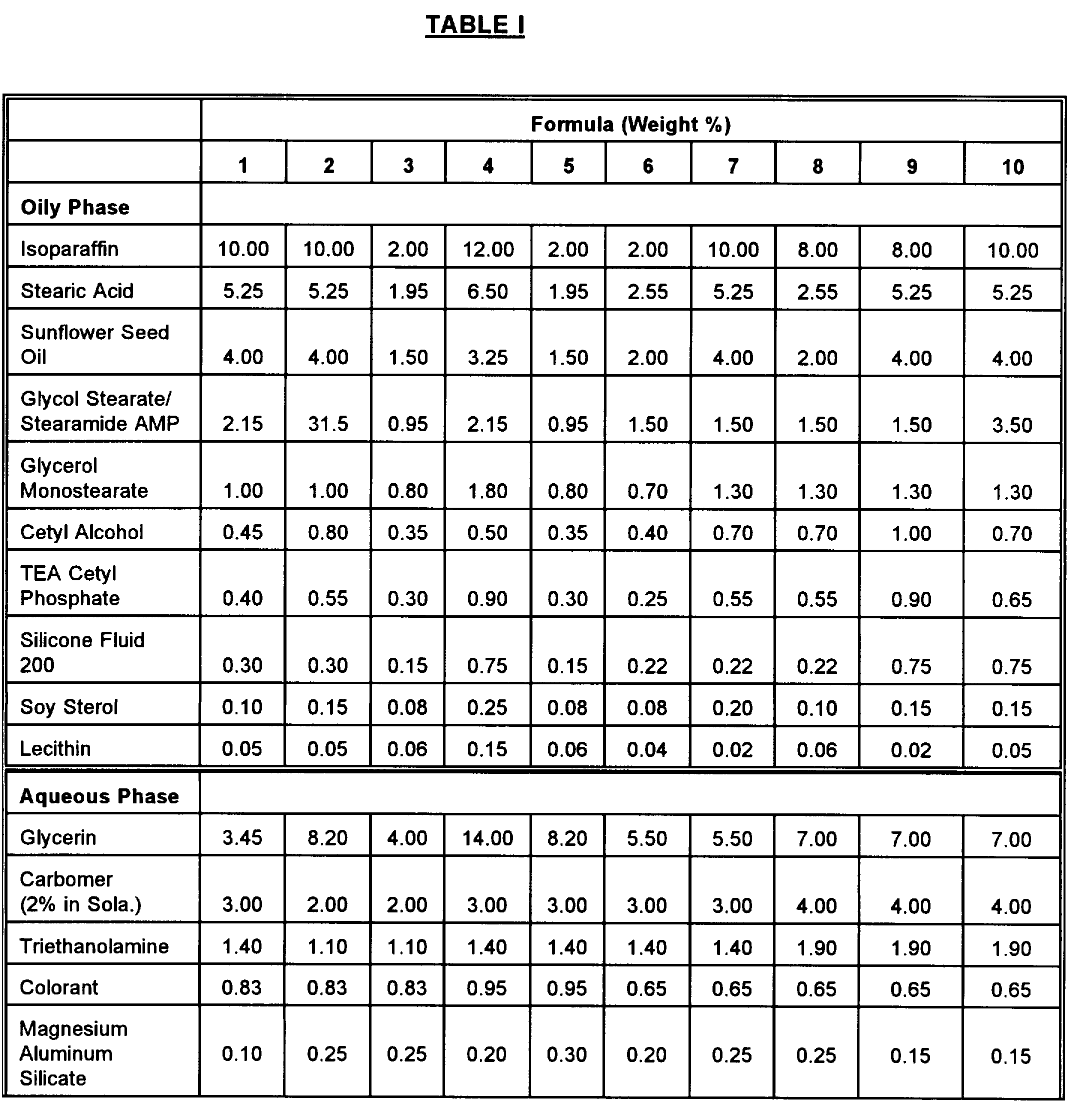

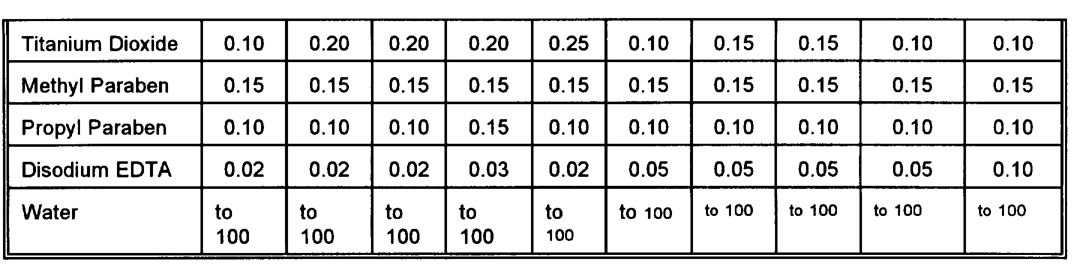

### EXAMPLES 11-15

Further illustrations of formulations according to the present invention are described below.

**TABLE II**

| | **Formula (Weight %)** | | | | |
|---|---|---|---|---|---|
| | **11** | **12** | **13** | **14** | **15** |
| **Oily Phase** | | | | | |
| Isoparaffin | 5.00 | 10.00 | 10.00 | 20.00 | 20.00 |
| Oleic Acid | 4.25 | 4.25 | 4.25 | 8.15 | 8.15 |
| Palmitic Acid | -- | 2.00 | 2.00 | -- | -- |
| Borage Seed Oil | 1.00 | 1.00 | -- | -- | -- |
| Sunflower Seed Oil | -- | -- | 2.00 | 4.00 | 4.00 |
| Glycerol Monoisostearate | 1.00 | 1.00 | -- | -- | -- |
| Sorbitan Monooleate | -- | -- | 1.50 | 1.50 | 2.00 |
| Myristyl Alcohol | 0.55 | 0.55 | 0.55 | 0.80 | 0.80 |
| DEA Stearyl Phosphate | 0.55 | 0.55 | 0.55 | 0.80 | 0.80 |
| Dimethicone | 0.40 | 0.40 | 0.40 | 0.75 | 0.75 |
| Dimethicone Copolyol | 0.30 | 0.30 | 0.30 | 0.20 | 0.20 |
| Soy Sterol | 0.15 | -- | -- | 0.10 | 0.10 |
| Cholesterol | 0.05 | 0.05 | 0.05 | 0.05 | 0.05 |
| Lecithin | 0.05 | 0.05 | 0.05 | 0.05 | 0.05 |
| **Aqueous Phase** | | | | | |
| Propylene Glycol | 15.00 | 10.00 | 10.00 | 5.00 | 5.00 |
| Xanthan Gum | 3.00 | -- | 3.00 | -- | 3.00 |
| Sodium Carboxymethyl Cellulose | -- | 3.00 | -- | 3.00 | -- |
| Colorant | 0.50 | 0.50 | 0.50 | 0.50 | 0.50 |
| Titanium Dioxide | 0.20 | 0.20 | 0.30 | -- | 0.30 |
| Zinc Oxide | 0.10 | 0.10 | -- | 0.30 | -- |
| DMDM Hydantoin | 0.15 | 0.15 | 0.15 | 0.15 | 0.15 |
| Water | to 100 | to 100 | to 100 | to 100 | to 100 |

### EXAMPLE 16

This example establishes the effectiveness of low levels of sterol/lecithin with respect to imparting a conditioned skinfeel to compositions according to the present invention.

**TABLE III**

| **INGREDIENT** | **FORMULA** | | | | |
|---|---|---|---|---|---|
| | **A** | **B** | **C** | **D** | **E** |
| Sunflower Seed Oil | 2.5 | 2.5 | 2.5 | 2.5 | 2.5 |
| Cetyl Alcohol | 2.5 | 2.5 | 2.5 | 2.5 | 2.5 |
| Glyceryl Monostearate | 1 | 1 | 1 | 1 | 1 |
| Amphisol | 2 | 2 | 2 | 2 | 2 |
| Isoparaffin | 2 | 2 | 2 | 2 | 2 |
| Water | 90 | 89.8 | 88 | 89 | 89.5 |
| Soy Sterol | 0 | 0.1 | 1 | 0.5 | 0.25 |
| Lecithin | 0 | 0.1 | 1 | 0.5 | 0.25 |
| Emulsion Formation | yes | yes | no | no | yes, grainy |
| Vis (#4 Spindle, 60 RPM, LVT @ 25°C) | 45 | 61 | -- | -- | 37 |
| pH | 6.1 | 6.05 | -- | -- | 5.96 |
| Conditioned Skinfeel | no | borderline | -- | -- | yes |

Based on the results in Table III, it is seen that relatively high levels of sterol/lecithin as in Formula C interfered with emulsion formation. Formulas B and E permit emulsion formation but also provide a conditioned skinfeel, especially as compared to Formula A having no sterol/lecithin present.

### EXAMPLE 17

A clinical trial was conducted on a composition according to the present invention to establish the effect of small amounts (0.12%) of sterol/lecithin. The formulation illustrated under Example 6 was utilized for the panel evaluation. For comparison purposes, a control was also tested having a formula identical to Example 6, except for the absence of sterol/lecithin and 2% sunflower seed oil (vehicle for the lipid mixture).

The clinical was conducted with women panelists ranging in age from 25 to 59 years who were users of mass marketed lotions. The panelists were divided into two groups of 40-50 persons each. One group was provided with the control and the other with the experimental formula. They were instructed to use the product at least once a day in place of their regular brand of hand and body lotion. After a 10-day usage period, the panelists were questioned with respect to various aspects of their product. They were requested to rate certain attributes as either excellent, very good, good, fair or poor. For example, when questioned about the attribute of absorbing into the skin, 50% of the respondents using the control rated the control as either excellent or very good. Of the respondents using the Example 6 product, 62% rated the absorbing into the skin attribute as either excellent or very good. Table IV summarizes results from the questionnaire.

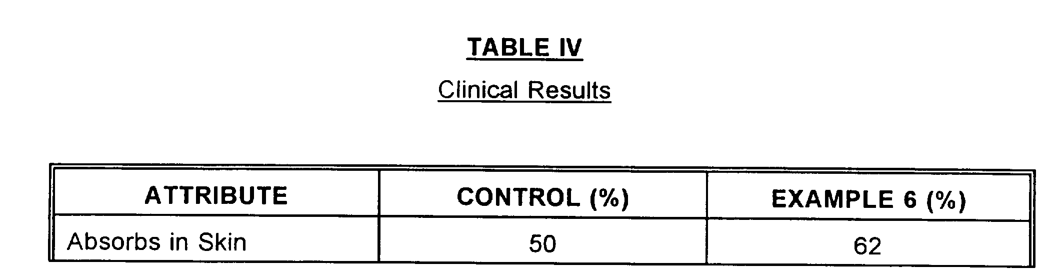

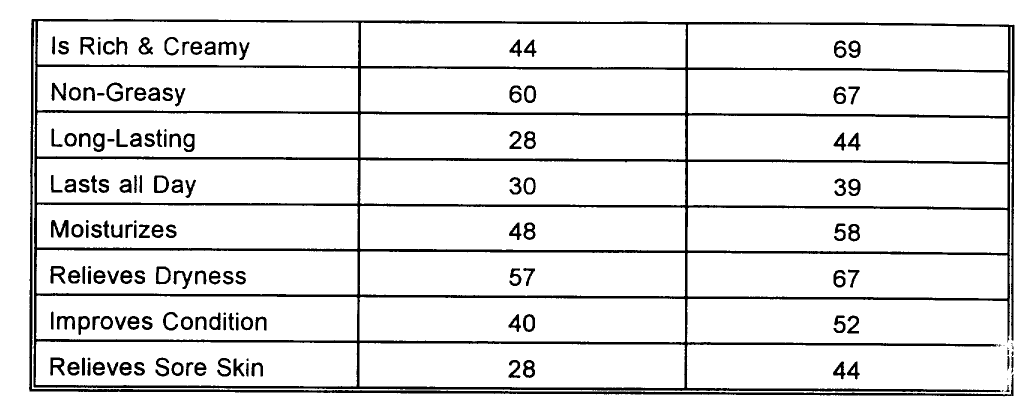

From results of the Table it is seen that the Example 6 product scored higher than the control with respect to the attributes: absorbs in skin, rich & creamy, non-greasy, long-lasting, lasts all day, moisturizes, relieves dryness, improves condition and relieves sore skin. These results were quite significant, especially in view of the very low levels of lipid incorporated into Example 6 versus the control.

The foregoing description and Examples illustrate select embodiments of the present invention. In light thereof, various modifications will be suggested to one skilled in the art, all of which are within the purview of this invention.

## Claims

1. An oil and water cosmetic emulsion comprising:
(i) from about 0.1 to 20% of isoparaffin;
(ii) from about 0.001 to 0.8% of sterol; and
(iii) from about 0.001 to 0.8% of phosphatide.

2. A composition according to claim 1 further comprising from about 0.01 to about 10% of C₈-C₂₂ alkyl phosphate salt.

3. A composition according to claim 1 wherein the sterol selected from the group consisting of cholesterol, stigmasterol, sitosterol, ergosterol and combinations thereof.

4. A composition according to claim 1 further comprising from about 0.1 to about 20% of C₁₆-C₂₂ alkanoic triglyceride.

5. A composition according to claim 4 wherein the triglyceride is sunflower seed oil.

6. A composition according to claim 1 wherein water is present in an amount from about 50 to about 95%.

7. A composition according to claim 1 wherein the amount of sterol present ranges from about 0.1 to about 0.3% by weight of the total composition.

8. A composition according to claim 1 wherein the amount of phosphatide ranges from about 0.1 to about 0.3% by weight of the total composition.

9. A composition according to claim 1 wherein the phosphatide is lecithin.
